Europäisches Patentamt

(19) European Patent Office

Office européen des brevets

(11) Publication number: **0 345 926 B1**

## (12) EUROPEAN PATENT SPECIFICATION

(45) Date of publication of patent specification:
07.08.91 Bulletin 91/32

(51) Int. Cl.⁵: **A61K 31/155**, A61K 9/08,
A61K 9/72

(21) Application number: 89303639.2

(22) Date of filing: 12.04.89

(54) Pharmaceutical compositions containing pentamidine.

(30) Priority: 25.04.88 US 185463

(43) Date of publication of application:
13.12.89 Bulletin 89/50

(45) Publication of the grant of the patent:
07.08.91 Bulletin 91/32

(84) Designated Contracting States:
AT BE CH DE ES FR GB GR IT LI LU NL SE

(56) References cited:
EP-A- 0 292 100
DE-A- 3 533 494
AMERICAN JOURNAL OF HOSPITAL PHAR-
MACY, vol. 43, no. 6, June 1986, pages
1486-1488, American Society of Hospital Phar-
macists, Bethesda, MD, US; N.C. DE et al.:
"Stability of pentamidine isethionate in 5 dext-
rose and 0.9 sodium chloride injections"

(73) Proprietor: FISONS CORPORATION
Jefferson Road
Rochester, NY 14603 (US)

(72) Inventor: Anaebonam, Aloysius
245 Loden Lane
Rochester, NY 14623 (US)
Inventor: Clemente, Emmett
23 Loading Place Road
Manchester, MA 01944 (US)
Inventor: Devlin, Theresa
9 Oak Square Avenue
Brighton, MA 02135 (US)
Inventor: Rindgen, Diane
48 Jamaica Street, Apartment 1
Jamaica Plain, MA 02130 (US)

(74) Representative: Wright, Robert Gordon McRae
FISONS plc 12 Derby Road
Loughborough Leicestershire LE11 0BB (GB)

EP 0 345 926 B1

EP 0 345 926 B1

**Description**

This invention relates to pharmaceutical compositions, in particular to aqueous solutions of pentamidine.

1,5-Di(4-amidinophenoxy)pentane, which is generically known as pentamidine, has for many years been known for use as a pharmaceutical, in particular for the treatment of the early stages of African trypanosomiasis ('sleeping sickness'). Pentamidine has also been found to be effective in the treatment of pneumo-cystis carinii pneumonia (PCP), a condition which is commonly contracted by patients with acquired immuno-deficiency syndrome (AIDS) and also by cancer and organ transplant patients. It has been estimated that some 65% of AIDS patients develop PCP. Amongst such patients the condition is life-threatening.

PCP infection in AIDS patients using pentamidine has been by intravenous infusion or intramuscular injection although this treatment is often accompanied by severe side-effects, eg hypotension, renal failure and hypoglycaemia. More recently, there has been reported (Abstracts of the Annual Meeting of the American Society of Microbiology 86, 14 (1986)) a method for the prevention of PCP which comprises the administration by inhalation of a nebulised aqueous solution of pentamidine.

A problem which has hitherto been encountered with the use of aqueous solutions of pentamidine is that, as described in, for example, Martindale's "The Extra Pharmacopoeia", 26th Edition, page 1862 (The Pharmaceutical Press, London), such solutions, which have a pH in the range 4.5 to 6.5, deteriorate on storage and must therefore be made up immediately prior to use.

We have now surprisingly found that the stability of aqueous solutions of pentamidine can be greatly improved by adjusting the pH of the solution downwardly.

Thus, according to the invention we provide an aqueous solution containing, as active ingredient, pentamidine or a pharmaceutically acceptable salt thereof, the solution having a pH of less than 4.5.

The solution according to the invention is advantageous in that it shows a markedly reduced tendency to deteriorate on storage than do known solutions of the active ingredient and hence can be stored for considerably longer times. This eliminates the need for the solution to be made up immediately prior to use and enables the solution to be supplied in ready-to-use form, thereby greatly increasing the convenience to the user.

The concentration of active ingredient in the solution may be from 0.1 to 10% w/v (measured as the free base). The concentration of active ingredient is, however, preferably less than 5%, and more preferably less than 3% eg 1% or 2% w/v.

Pharmaceutically acceptable salts of pentamidine which may be used include the isethionate, the naphthoate and the mesylate. We particularly prefer to use the isethionate.

The pH of the solution is preferably less than 4.0, more preferably less than 3.5, and especially less than 3.0.

The pH of the solution may be greater than 1.0, is preferably greater than 1.5, more preferably greater than 2.0 and especially greater than 2.5.

The pH of the solution may be adjusted by the addition of a pharmaceutically acceptable acid. Pharmaceutically acceptable acids which may be used include organic acids, eg acetic acid, and, more preferably, mineral acids, eg hydrochloric acid and sulphuric acid. A particularly preferred acid which may be used to adjust the pH is hydrochloric acid.

The solution may be buffered or, more preferably, non-buffered. Buffering agents which may be used will be readily apparent to those skilled in the art but, by way of example, the following may be mentioned : sodium dihydrogen orthophosphate (sodium acid phosphate BP), di-sodium hydrogen phosphate (sodium phosphate BP), sodium citrate/citric acid, and boric acid/sodium borate.

The solution may be made isotonic with physiological fluids by the incorporation of a suitable tonicity agent eg sodium chloride. The solution may typically contain from about 0.1 to 1.0, more typically 0.5 to 1.0% w/v sodium chloride. We have found, however, that the stability of solutions containing sodium chloride is not as great as that of solutions containing no sodium chloride, particularly when the solutions are stored at reduced temperature (eg 4°C).

Other excipients which may be present in the solution include chelating or sequestering agents. Suitable chelating or sequestering agents include sodium carboxymethyl cellulose, citric, tartaric and phosphoric acids, and amino carboxylate compounds. The preferred chelating agent, however, is ethylenediamine tetraacetic acid or a salt thereof, especially the disodium salt.

The proportion and concentration of buffers, if included, and other excipients may be varied within fairly wide ranges, provided the resulting solution is stable and non-irritant when applied to the appropriate tissues. The maximum total concentration of excipients and buffers is preferably less than 5% w/v and more preferably less than 2% w/v.

The solutions may be made up, for example, by dissolving the active ingredient and excipients (if included) in freshly distilled water, adjusting the pH if necessary, making the solution up to the desired volume with distilled

2

water, stirring and then sterilising. Sterilisation is preferably performed by sterile filtration into a previously sterilised container.

The solution is preferably made up at a temperature of from about 10 to 50°C, for example at room temperature.

We have found that when the solution contains sodium chloride, the solution is best prepared by dissolving the sodium chloride and the active ingredient in separate aliquots of distilled water and then mixing the two solutions. If this procedure is not followed, eg if the active ingredient and the sodium chloride are added as solids to the distilled water or if solid sodium chloride is added to a solution of the active ingredient, then a precipitate may be formed which can only be dissolved by protracted stirring.

The solution may be put up in unit dosage form, in which case preservatives may be incorporated, but are generally not necessary. Alternatively the solution may be put up in multi-dose form. In general it will be necessary to incorporate one or more preservatives into multi-dose solutions to ensure that the solution remains sterile after initial use.

The preferred preservative for solutions for inhalation is chlorbutol. The concentration of preservative should be such as to ensure effective preservation of the solution ie such that bacterial growth in the solution is inhibited. For most preservatives the concentration will typically lie in the range 0.001 to 0.1% w/v. However, in the case of chlorbutol acceptable concentrations are greater than 0.25% but less than 0.6% w/w ie the concentration of chlorbutol is 0.25 to 0.6%, preferably 0.3 to 0.55% eg 0.5% w/w.

Unit doses of the solutions of the invention for use in nebulisers may be packed in glass or plastics ampoules which are broken open immediately prior to use.

Multi-dose solutions may be packaged in volumes of 5 to 300 ml. Preferred volumes for inhalation compositions include 60, 120 and 240 ml.

We prefer multi-dose solutions to be packaged such that unit volumes of the solution to be administered can be accurately dispensed. The solution may, for example, be packaged in a flexible-walled container provided with a cap to receive the unit volume.

The required dose of active ingredient to be administered will vary with, amongst other factors, the severity of the condition being treated, and will depend on whether the treatment is remedial or prophylactic.

For administration by intravenous infusion or intramuscular injection (generally for remedial treatment), a dose of about 4 mg/kg/day is administered for 14 to 21 days. For administration by these routes the solution of the invention would typically be diluted immediately before use.

For administration to the lungs by nebulisation, a dose of from about 1 to 200 mg, preferably from 20 to 80 mg and especially about 40 mg is appropriate for prophylactic treatment. Each dose is typically administered weekly for 4 to 6 weeks and thereafter biweekly. For remedial treatment of PCP infection, more frequent dosage may be called for, eg 1 to 8 times (preferably 4 times) daily. This higher dosage regime may be maintained for as long as the infection persists (typically 7 to 21 days).

The solution may be administered directly to the lung by nebulisation without dilution, particularly where (as in the likely dosing schedule described above) nebulisation occurs only once a week or once every two weeks. Should more frequent dosing be required, or where the solution is administered by another route, eg by infusion, it may be necessary to adjust the pH of the solution to a higher value prior to administration. This may be achieved by simple dilution or, more efficiently, by mixing with a second solution, perhaps of alkaline pH, such that the final solution has the desired pH. The second solution may conveniently be a saline solution with the pH increased by the incorporation of, for example, sodium hydroxide. Similarly, a hypotonic solution may be rendered isotonic immediately prior to use by the addition of an aliquot of saline solution.

According to another aspect of the invention there is provided a pack comprising a first solution containing, as active ingredient, pentamidine or a pharmaceutically acceptable salt thereof, the solution having a pH of less than 4.5, and a second solution which when mixed with the first solution gives a final solution of pH 4.5 to 7.0.

The first solution or, more preferably, the second solution may contain sodium chloride at such a concentration that the final solution is isotonic.

Preferably both first and second solutions are packaged in glass or plastics ampoules. The volumes of the two solutions may be the same or different. For example, the first solution may be 2 ml of a solution containing 3% w/v pentamidine isethionate and having a pH of about 3.0, and the second solution may be 1 ml of a saline solution with its pH adjusted by the incorporation of sodium hydroxide.

According to a further aspect of the invention, there is provided the use of pentamidine, or a pharmaceutically acceptable salt thereof, as active ingredient in the manufacture of a pharmaceutical composition comprising an aqueous solution of the active ingredient, the solution having a pH of less than 4.5.

The invention is illustrated, but in no way limited, by the following Examples.

## Example 1

### Non-preserved nebuliser solution

| | |
|---|---|
| Pentamidine isethionate | 1.0 % w/v |
| Hydrochloric acid | q.s. |
| Purified water | to 100 |

Pentamidine isethionate (10 g) was dissolved in purified water (900 ml). The pH of the solution was adjusted to between 2.5 and 3.0 by addition of hydrochloric acid. The volume was made up to 1000 ml with purified water. The solution was sterile-filled into glass ampoules which were then sealed.

## Example 2

### Non-preserved isotonic nebuliser solution

| | |
|---|---|
| Pentamidine isethionate | 1.0 % w/v |
| Sodium chloride | 0.79 |
| Hydrochloric acid | q.s. |
| Purified water | to 100 |

Pentamidine isethionate (10 g) was dissolved in purified water (800 ml). The pH of the solution was adjusted to between 2.5 and 3.0 by addition of hydrochloric acid. Sodium chloride (7.9 g) was dissolved in a second aliquot (100 ml) of purified water and the resulting solution mixed with the pH-adjusted pentamidine isethionate solution. The volume was made up to 1000 ml with purified water.
The solution was sterile-filled into glass ampoules which were then sealed.

## Example 3

### Preserved isotonic nebuliser solution

| | |
|---|---|
| Pentamidine isethionate | 1.0 % w/v |
| Sodium chloride | 0.79 |
| Chlorbutol | 0.5 |
| Hydrochloric acid | q.s. |
| Purified water | to 100 |

Chlorbutol (5 g) was dissolved in purified water (800 ml). Pentamidine isethionate (10 g) was added and the pH of the solution adjusted to between 2.5 and 3.0 by addition of hydrochloric acid. Sodium chloride (7.9 g) was dissolved in a second aliquot (100 ml) of purified water and the resulting solution mixied with the pH-adjusted pentamidine isethionate solution. The volume was then made up to 1000 ml with purified water.
The solution was filled into polyethylene bottles of 120 ml capacity.

Example 4

Measurement of Stability of Solutions at Various pH Values Method

Solutions of pentamidine isethionate with a concentration of 10 mg/ml were prepared at various values of pH (the pH was adjusted by the incorporation into the solutions of hydrochloric acid). The solutions were sealed into glass ampoules.

The solutions were heated at 80°C for periods of 20-21.5 hours. Some samples were also heated for 44 hours. After heating, the concentration of pentamidine isethionate related impurities (expressed as a percentage) was determined by HPLC.

Results

| pH | % Impurities | |
|---|---|---|
| | 20-21.5 hours | 44 hours |
| 5.60 | 12.10 | --- |
| 5.41 | 14.10 | 19.30 |
| 3.99 | 0.89 | --- |
| 2.74 | 0.28 | 0.75 |
| 2.11 | 0.36 | 0.67 |

**Claims**

**Claims for the following Contracting States : AT BE CH DE FR GB IT LI LU NL SE**

1. An aqueous solution containing, as active ingredient, pentamidine or a pharmaceutically acceptable salt thereof, the solution having a pH of less than 4.5.

2. A solution according to Claim 1, wherein the concentration of active ingredient in the solution is from 0.1 to 5% w/v.

3. A solution according to Claim 1 or Claim 2, wherein the pH of the solution is greater than 2.0.

4. A solution according to any one of the preceding claims, wherein the pH of the solution is less than 4.0.

5. A solution according to any one of the preceding claims, wherein the pH of the solution is less than 3.5.

6. A solution according to any one of the preceding claims, wherein the active ingredient is pentamidine isethionate.

7. A solution according to any one of the preceding claims, which comprises hydrochloric acid.

8. A pack comprising a first solution containing, as active ingredient, pentamidine or a pharmaceutically acceptable salt thereof, the solution having a pH of less than 4.5, and a second solution which when mixed with the first solution gives a final solution of pH 4.5 to 7.0.

9. A pack according to Claim 8, wherein the second solution contains sodium chloride at such a concentration that the final solution is isotonic.

10. A process for the preparation of an aqueous solution containing pentamidine or a pharmaceutically acceptable salt thereof, as active ingredient, and sodium chloride, and having a pH of less than 4.5, which process comprises mixing an aqueous solution of the active ingredient and an aqueous solution of sodium chloride.

EP 0 345 926 B1

**Claims for the following Contracting States : ES and GR**

1. A process for the preparation of an aqueous solution containing, as active ingredient, pentamidine or a pharmaceutically acceptable salt thereof, the solution having a pH of less than 4.5, which process comprises mixing the ingredients.

2. A process according to Claim 1, wherein the concentration of active ingredient in the solution is from 0.1 to 5% w/v.

3. A process according to Claim 1 or Claim 2, wherein the pH of the solution is greater than 2.0.

4. A process according to any one of the preceding claims, wherein the pH of the solution is less than 4.0.

5. A process according to any one of the preceding claims, wherein the pH of the solution is less than 3.5.

6. A process according to any one of the preceding claims, wherein the active ingredient is pentamidine isethionate.

7. A process according to any one of the preceding claims, which comprises hydrochloric acid.

8. A process for the preparation of an aqueous solution containing pentamidine or a pharmaceutically acceptable salt thereof, as active ingredient, and sodium chloride, and having a pH of less than 4.5, which process comprises mixing an aqueous solution of the active ingredient and an aqueous solution of sodium chloride.

9. A pack comprising a first solution containing, as active ingredient, pentamidine or a pharmaceutically acceptable salt thereof, the solution having a pH of less than 4.5, and a second solution which when mixed with the first solution gives a final solution of pH 4.5 to 7.0.

10. A pack according to Claim 8, wherein the second solution contains sodium chloride at such a concentration that the final solution is isotonic.


**Patentansprüche**


**Patentansprüche für folgende Vertrasstaaten : AT, BE, CH, DE, FR, GB, IT, LI, LU, NL, SE**

1. Wässerige Lösung enthaltend als aktiven Inhaltsstoff Pentamidin oder ein pharmazeutisch annehmbares Salz hievon, wobei die Lösung einen pH von weniger als 4,5 aufweist.

2. Lösung nach Anspruch 1, worin die Konzentration an aktivem Inhaltsstoff in der Lösung 0,1 bis 5% G/V beträgt.

3. Lösung nach Anspruch 1 oder 2, worin der pH der Lösung größer als 2,0 ist.

4. Lösung nach einem der vorhergehenden Ansprüche, worin der pH der Lösung kleiner als 4,0 ist.

5. Lösung nach einem der vorhergehenden Ansprüche, worin der pH der Lösung kleiner als 3,5 ist.

6. Lösung nach einem der vorhergehenden Ansprüche, worin der aktive Inhaltsstoff Pentamidinisäthionat ist.

7. Lösung nach einem der vorhergehenden Ansprüche, die Chlorwasserstoffsäure umfaßt.

8. Packung, umfassend eine erste Lösung mit einem pH von weniger als 4,5, enthaltend als aktiven Inhaltsstoff Pentamidin oder ein pharmazeutisch annehmbares Salz hievon und eine zweite Lösung, die, wenn sie mit der ersten Lösung gemischt wird, eine Endlösung mit pH 4,5 bis 7,0 ergibt.

9. Packung nach Anspruch 8, worin die zweite Lösung Natriumchlorid in einer solchen Konzentration enthält, daß die Endlösung isotonisch ist.

10. Verfahren zur Herstellung einer wässerigen Lösung, die Pentamidin oder ein pharmazeutisch annehmbares Salz hievon, als aktiven Inhaltsstoff und Natriumchlorid enthält und einen pH von weniger als 4,5 aufweist, wobei das Verfahren das Mischen einer wässerigen Lösung des aktiven Inhaltsstoffes mit einer wässerigen Lösung von Natriumchlorid umfaßt.

**Patentansprüche für folgende Vertrasstaaten : ES und GR**

1. Verfahren zur Herstellung einer wässerigen Lösung enthaltend als aktiven Inhaltsstoff Pentamidin oder ein pharmazeutisch annehmbares Salz hievon, wobei die Lösung einen pH von weniger als 4,5 aufweist, welches Verfahren das Mischen der Inhaltsstoffe umfaßt.

2. Verfahren nach Anspruch 1, wobei die Konzentration an aktivem Inhaltsstoff in der Lösung 0,1 bis 5% G/V beträgt.

3. Verfahren nach Anspruch 1 oder 2, wobei der pH der Lösung größer als 2,0 ist.

4. Verfahren nach einem der vorhergehenden Ansprüche, wobei der pH der Lösung kleiner als 4,0 ist.

5. Verfahren nach einem der vorhergehenden Ansprüche, wobei der pH der Lösung kleiner als 3,5 ist.

6. Verfahren nach einem der vorhergehenden Ansprüche, wobei der aktive Inhaltsstoff Pentamidinisäthionat ist.

7. Verfahren nach einem der vorhergehenden Ansprüche, das Chlorwasserstoffsäure umfaßt.

8. Verfahren zur Herstellung einer wässerigen Lösung, die Pentamidin oder ein pharmazeutisch annehmbares Salz hievon als aktiven Inhaltsstoff und Natriumchlorid enthält und einen pH von weniger als 4,5 aufweist, wobei das Verfahren das Mischen einer wässerigen Lösung des aktiven Inhaltsstoffes mit einer wässerigen Lösung von Natriumchlorid umfaßt.

9. Packung, umfassend eine erste Lösung mit einem pH von weniger als 4,5, enthaltend als aktiven Inhaltsstoff Pentamidin, oder ein pharmazeutisch annehmbares Salz hievon, und eine zweite Lösung die, wenn sie mit der ersten Lösung gemischt wird, eine Endlösung mit pH 4,5 bis 7,0 ergibt.

10. Verpackung nach Anspruch 9, worin die zweite Lösung Natriumchlorid in einer solchen Konzentration enthält, daß die Endlösung isotonisch ist.

## Revendications

### Revendications pour les Etats contractants suivants : AT, BE, CH, DE, GB, IT, LI, LU, NL, SE, FR

1. Solution aqueuse contenant, comme constituant actif, de la pentamidine ou un sel pharmaceutiquement acceptable de celle-ci, la solution ayant un pH inférieur à 4,5.

2. Solution suivant la revendication 1, dans laquelle la concentration du constituant actif dans la solution est de 0,1 à 5,0% p/v.

3. Solution suivant la revendication 1 ou 2, dans laquelle le pH de la solution est supérieur à 2,0.

4. Solution suivant l'une quelconque des revendications précédentes, dans laquelle le pH de la solution est inférieur à 4,0.

5. Solution suivant l'une quelconque des revendications précédentes, dans laquelle le pH de la solution est inférieur à 3,5.

6. Solution suivant l'une quelconque des revendications précédentes, dans laquelle le constituant actif est l'iséthionate de pentamidine.

7. Solution suivant l'une quelconque des revendications précédentes, qui comprend de l'acide chlorhydrique.

8. Conditionnement comprenant une première solution contenant, comme constituant actif, de la pentamidine ou un sel pharmaceutiquement acceptable de celui-ci, la solution ayant un pH inférieur à 4,5, et une deuxième solution qui, lorsqu'elle est mélangée avec la première solution, donne une solution finale d'un pH de 4,5 à 7,0.

9. Conditionnement suivant la revendication 8, dans lequel la deuxième solution contient du chlorure de sodium en une concentration telle que la solution finale soit isotonique.

10. Procédé de préparation d'une solution aqueuse contenant de la pentamidine ou un sel pharmaceutiquement acceptable de celle-ci comme constituant actif, et du chlorure de sodium, et ayant un pH inférieur à 4,5, lequel procédé comprend le mélange d'une solution aqueuse du constituant actif avec une solution aqueuse de chlorure de sodium.

### Revendications pour les Etats contractants suivants : ES et GR

1. Procédé de préparation d'une solution aqueuse contenant, comme constituant actif, de la pentamidine ou un sel pharmaceutiquement acceptable de celle-ci, la solution ayant un pH inférieur à 4,5, lequel procédé comprend le mélange des constituants.

2. Procédé suivant la revendication 1, dans lequel la concentration du constituant actif dans la solution est de 0,1 à 5,0% p/v.

3. Procédé suivant la revendication 1 ou 2, dans lequel le pH de la solution est supérieur à 2,0.

4. Procédé suivant l'une quelconque des revendications précédentes, dans lequel le pH de la solution est inférieur à 4,0.

5. Procédé suivant l'une quelconque des revendications précédentes, dans lequel le pH de la solution est inférieur à 3,5.

6. Procédé suivant l'une quelconque des revendications précédentes, dans lequel le constituant actif est l'iséthionate de pentamidine.

7. Procédé suivant l'une quelconque des revendications précédentes, qui comprend de l'acide chlorhydrique.

8. Procédé de préparation d'une solution aqueuse contenant de la pentamidine ou un sel pharmaceutiquement acceptable de celle-ci comme constituant actif, et du chlorure de sodium, et ayant un pH inférieur à 4,5, lequel procédé comprend le mélange d'une solution aqueuse du constituant actif avec une solution aqueuse de chlorure de sodium.

9. Conditionnement comprenant une première solution contenant, comme constituant actif, de la pentamidine ou un sel pharmaceutiquement acceptable de celui-ci, la solution ayant un pH inférieur à 4,5, et une deuxième solution qui, lorsqu'elle est mélangée avec la première solution, donne une solution finale d'un pH de 4,5 à 7,0.

10. Conditionnement suivant la revendication 9, dans lequel la deuxième solution contient du chlorure de sodium en une concentration telle que la solution finale soit isotonique.